# EUROPÄISCHE PATENTANMELDUNG

(11) **EP 0 573 405 A1**
(43) Veröffentlichungstag der Anmeldung: **08.12.1993**
(21) Anmeldenummer: 93890111.3
(22) Anmeldetag: 28.05.1993
(51) Int. Cl.: A61M 1/02

(54) **Mehrfachblutbeutelsystem zur sterilen Behandlung von menschlichen Blutkonserven**

(30) Priorität: 01.06.1992 AT 1134/92
(71) Anmelder: Öko Wien Umweltschutzprojektentwicklungs- und managementgesellschaft m.b.H., A-1040 Wien (AT)
(72) Erfinder: Wagner, Andrea, A-1170 Wien (AT)
(74) Vertreter: Itze, Peter, Dipl.-Ing.

(57) **Zusammenfassung**

Die Erfindung betrifft ein Mehrfachbeutelsystem zur sterilen Behandlung von menschlichen Blutkonserven, mit dessen Hilfe das Blut filtriert und gewaschen werden kann, und wobei wenigstens ein Auffangbeutel für nicht benötigte Bestandteile mit dem Blutsammelbeutel verbunden ist. Hiebei ist der Blutsammelbeutel 1 an einem Ende einer Verteilerleitung 3 und ein Vorratsbeutel 4 für die Waschflüssigkeit an deren anderem Ende steril angeschlossen, wobei die Verteilerleitung 3 vor dem Vorratsbeutel 4, vorzugsweise mittels einer Klemme 5 od.dgl, abschließbar ist, und wobei in bekannter Weise von der Verteilerleitung 3 wenigstens zwei parallel verlaufende Abzweigleitungen 6, 7 ausgehen, wobei eine davon zu einem Auffangbeutel 11 für verbrauchte Waschflüssigkeit und die andere zu einem Aufnahmebeutel 10 für gewaschenes Erythrozytenkonzentrat führt, und wobei weiters in bekannter Weise in der Verteilerleitung 3 dem Blutsammelbeutel 1 ein Leukozytenfilter 2 nachgeschaltet ist.

## Beschreibung

Die Erfindung bezieht sich auf ein Mehrfachbeutelsystem zur sterilen Behandlung von menschlichen Blutkonserven, mit dessen Hilfe das Blut filtriert und gewaschen werden kann, und wobei wenigstens ein Auffangbeutel für nicht benötigte Bestandteile mit dem Blutsammelbeutel verbunden ist.

Bei bekannten Ausbildungen dieser Art ist der Blutsammelbeutel, in welchem das dem Spender abgezapfte Blut direkt aufgenommen wird, mit einer sich auf zwei Leitungen verzweigenden Leitung verbunden, die jeweils steril mit einem Beutel verbunden sind. Weiters ist der Blutsammelbeutel noch mit einer weiteren Leitung verbunden, welche zu einem Lösungsmittelbeutel führt, wobei in dieser Leitung ein Filter zwischengeschaltet ist. Bei dieser bekannten Ausbildung wird nach Filtration das Blutplasma in den Lösungsmittelbeutel hinübergeführt und bei der Rückführung durch das in der Leitung eingeschaltete Filter gereinigt. Das Plasma wird dann in einen der an der sich verzweigenden Leitung angeschlossenen Beutel abgefüllt und so von dem Erythrozytenkonzentrat getrennt. Mit dieser bekannten Ausbildung ist allerdings eine Waschung des Erythrozytenkonzentrats schwer möglich, da die eigentliche Behandlung direkt in dem Blutsammelbeutel bzw. in dem Lösungsmittelbeutel erfolgt.

Um bisher eine Waschung des Erythrozytenkonzentrats mit Kochsalzlösung zu ermöglichen, bei welcher Eiweiß und Kalium aus dem Erythrozytenkonzentrat herausgewaschen wird, war es bisher nötig, an den Blutsammelbeutel, also an die Rohkonserve, einen Leukozytenfilter anzuschweißen, mit welchem die Leukozyten zu 99 % und die Thrombozyten zu etwa 90 % herausgefiltert werden. Danach wird ein zweiter Reinigungsschritt durchgeführt, wobei die Konzentrate nach dem Filtrierungsprozeß geöffnet (angestochen) und zweimal mit physiologischer Kochsalzlösung nachgewaschen werden. Dieses Öffnen der Konserven (Anstechen) beinhaltet aber die Kontaminationsmöglichkeitdes Beutelinhalts, wodurch die Haltbarkeit dieser Konserven auf 6 Stunden reduziert ist.

Der Erfindung liegt nun die Aufgabe zugrunde, eine Einrichtung zu schaffen, mitwelcher in einem geschlossenen System, das dem Spender entnommene Blut von Leukozyten und Thrombozyten getrennt und das Erythrozytenkonzentrat mit Kochsalzlösung gewaschen werden kann, um Eiweiß und Kalium zu entfernen.

Erfindungsgemäß wird diese Aufgabe dadurch gelöst, daß an einem Ende der Verteilerleitung der Blutsammelbeutel und ein Vorratsbeutel für die Waschflüssigkeit an deren anderem Ende steril angeschlossen ist, wobei die Verteilerleitung vor dem Vorratsbeutel, vorzugsweise mittels einer Klemme od.dgl, abschließbar ist, und wobei in bekannter Weise von der Verteilerleitung wenigstens zwei parallelgeschaltete Abzweigleitungen ausgehen, daß eine davon zu einem Auffangbeutel für verbrauchte Waschflüssigkeit und die andere zu einem Aufnahmebeutel für gewaschenes Erythrozytenkonzentrat führt, und wobei weiters in bekannter Weise in der Verteilerleitung dem Blutsammelbeutel ein Leukozytenfilter nachgeschaltet ist. Durch eine solche Ausbildung ist es möglich, die Blutkonserve mittels einer Waschflüssigkeit, vorliegend ist dies physiologische Kochsalzlösung, zu waschen und die verbrauchte Waschflüssigkeit sowie das gereinigte Konzentrat gesondert aufzunehmen. Weiters erfolgt bereits vor der Behandlung eine Abtrennung der Leukozyten und Thrombozyten, sodaß die Waschflüssigkeit völlig für das Waschen der Erythrozyten bzw. der Erythrozytenlösung zur Verfügung steht.

Wie angeführt ist es an sich bekannt, von einer Verteilerleitung wenigstens zwei parallel verlaufende Abzweigleitungen ausgehen zu lassen, die zum Auffangbehälter führen. Bei dieser bekannten Ausbildung ist allerdings das Waschen von Erythrozytenkonzentraten nicht vorgesehen, sondern diese Ausbildung dient lediglich dazu, die nach einer Zentrifugation vorliegenden Phasen unterschiedlicher Dichte zu trennen.

Weiters ist es bereits bekannt in einer Verteilerleitung dem Blutsammelbeutel einen Leukozytenfilter nachzuschalten. Bei dieser bekannten Ausbildung ist ebenfalls ein Waschen von Erythrozytenkonzentrat nicht vorgesehen, sondern es wird das Erythrozytenkonzentrat lediglich mit einer Antikoagulationsflüssigkeit oder einer sonstigen zuzusetzenden Lösung versetzt. Eine Trennung von Waschwasser und Erythrozytenkonzentrat ist nicht vorgesehen.

Vorteilhafterweise kann von der Verteilerleitung wenigstens eine weitere Abzweigungsleitung ausgehen, an die ein oder mehrere weitere Auffangbeutel für verbrauchte Waschflüssigkeit oder Vorratsbeutel für weitere Behandlungsflüssigkeiten angeschlossen sind. Dies ermöglicht eine mehrfache Waschung bzw. auch eine Auftrennung der Waschflüssigkeiten in den einzelnen Waschgängen und ein getrenntes Auffangen dieser Waschflüssigkeiten.

Schließlich können die Auffangbeutel für verbrauchte Waschflüssigkeit und/oder Vorratsbeutel für weitere Behandlungsflüssigkeiten über an sich bekannte abbrechbare Ventile gegenüber der Verteilungsleitung abgeschlossen und durch Wegbrechen des Ventiles öffenbar sein, wodurch eine selektive Befüllung der einzelnen Beutel ermöglicht ist.

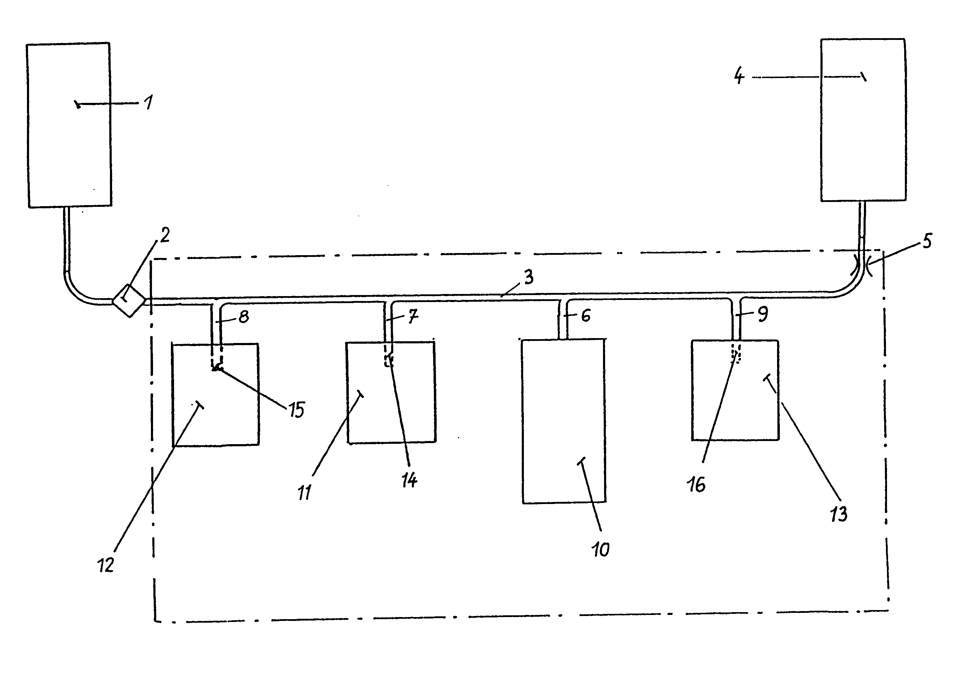

In der Zeichnung ist ein Ausführungsausführungsbeispiel des Erfindungsgegenstandes schematisch dargestellt.

Mit 1 ist der Blutsammelbeutel bezeichnet, in welchem das von einem Spender aufgenommene Blut gesammelt wird. Es handelt sich dabei um die sogenannte "Rohkonserve". Dieser Blutsammelbeutel 1 ist an eine Verteilerleitung 3 steril anschließbar, u.zw. anschweißbar, wobei in der Verteilerleitung 3 dem Blutsammelbeutel 1 nachgeschaltet ein Leukozytenfilter 2 vorgesehen ist. Am anderen Ende der Verteilerleitung ist ein mit physiologischer Kochsalzlösung gefüllter Beutel 4 gleichfalls steril an die Verteilerleitung 2 anschließbar. Diesem Beutel 4 unmittelbar nachgeschaltet ist ein Ventil 5 (vorzugsweise eine Klemme od.dgl.), mit welcher der Durchfluß der Natriumchloridlösung verhindert werden kann.

Von der Verteilerleitung 3 gehen Abzweigungsleitungen 6, 7, 8, 9 weg, von denen die erste Abzweigungsleitung 6 zu einem Erythrozytenkonzentratbeutel 10, die Abzweigungsleitungen 7, 8 zu Auffangbeutel für verbrauchte Waschflüssigkeit 11, 12, und die Abzweigleitung 9 zu einem Beutel für weitere Behandlungsflüssigkeit 13 (SAG-Mannitol) führt. Innerhalb derAuffangbeutel fürverbrauchte Waschflüssigkeit 11, 12 und dem Beutel für eine weitere Behandlungsflüssigkeit 13 sind abbrechbare Ventilt 14, 15, 16 vorgesehen, wobei durch Abbrechen dieser Ventile der Innenraum des jeweiligen Beutels mit der Verteilerleitung 3 verbindbar ist.

Für den Betrieb der Vorrichtung wird zunächst das im Blutsammelbeutel 1 gesammelte Rohblut durch den Leukozytenfilter hindurch in die Verteilerleitung 3 eingebracht und über die Abzweigung 6 in den Beutel 10 für Erythrozytenkonzentrat geleitet. Danach wird durch Öffnen des Ventils 5 ein Teil (ca. die Hälfte) der physiologischen Kochsalzlösung 4 in den Beutel 10 zusätzlich geleitet und das Erythrozytenkonzentrat mit der Waschlösung vermischt. Anschließend wird das gesamte Aggregat in einen Separator (Zentrifuge) eingebracht und zentrifugiert, wodurch sich die Erythrozyten am Boden des Beutels 10 ansammeln und der Überstand durch die Waschflüssigkeit und die ausgewaschenen Eiweiß- und Kaliumverbindung gebildet ist. Danach wird das abbrechbare Ventil 14 des Beutels 11 geöffnet und die überstehende Waschflüssigkeit über die Abzweigungsleitung 6, die Verbindungsleitung 3 und die Abzweigungsleitung 7 in den Beutel 11 eingebracht. Dieser wird dann z.B. durch Abschweißen verschlossen und entfernt. Darauffolgend wird die Klemme 5 geöffnet und die restliche Waschflüssigkeit aus dem Beutel 4 in den Beutel 10 eingebracht. Der Beutel 4 wird abgeschweißt und verworfen. Danach wird erneut das Erythrozytenkonzentrat mit der physiologischen Kochsalzlösung aus dem Beutel 4 vermischt und die ganze Einrichtung wieder in einem Separator zentrifugiert, sodaß sich die Erythrozyten wieder am Boden des Beutels 10 absetzen und der Überstand durch die verbrauchte Waschflüssigkeit gebildet ist. Es wird nun das Ventil 15 durch Abbrechen geöffnet und die überstehende Waschflüssigkeit in den Beutel 12 transferiert, wonach dann der Beutel 12, gleichfalls durch Abschweißen, verschlossen wird. Hierauf wird das Ventil 16 des Beutels 13 geöffnet und die darin befindliche weitere Behandlungsflüssigkeit, u.zw. im vorliegenden Fall SAG-Mannitollösung, in den Beutel 10 übergeführt und mit dem Erythrozytenkonzentrat gemischt. Danach ist die im Beutel 10 befindliche Erythrozytenkonzentratlösung, die mit dem SAG-Mannitol vermischt ist, gebrauchsfertig. In dieser Form kann das behandelte Erythrozytenkonzentrat bis zu 42 Tage gelagert werden.

Mit dem erfindungsgemäßen Mehrfachbeutelsystem zur Behandlung von Blutkonserven ist somit ein System geschaffen worden, mit welchem eine sterile Waschung des Erythrozytenkonzentrats ermöglicht ist, wodurch - wie schon angeführt - eine Erhöhung der Haltbarkeit von 6 Stunden auf 35, ja sogar bis 42 Tage, erreicht werden kann. Dadurch ist es möglich, daß für Operationen oder sonstige Behandlungen die Waschung des Erythrozytenkonzentrats nicht genau zeiteingeteilt erfolgen muß, sondern es können die nötigen Konserven bereits längere Zeit vorbereitet vorhanden sein und bei Bedarf entnommen werden.

Auch ist es möglich, nur Teile des Inhalts einer derartigen Konserve zu verwenden, wie es z.B. bei Bluttransfusionen von Neugeborenen usw. erforderlich ist, bei welchen ja früher aufgrund der kurzen Lagerfähigkeit, welche durch die unsterile Waschung hervorgerufen ist, oft zwei Drittel des Erythrozytenkonzentrats verworfen werden mußte. Erythrozytenkonzentrate werden auf Wunsch in dafür vorgesehene 150 ml Beutel aufgeteilt, z.B. eine gewaschene filtrierte Konserve ä 280 ml in fünf 150 ml Beutel ä 56 ml für je 1 Kind. Dies hat unter anderem den Vorteil, daß nur ein Spender für fünf Transfusionen benötigt wird, was eine Verringerung des Infektrisikos ergibt. Außerdem ist nur 1 HLA (Gewebstyp) notwendig. Es wird damit auch eine Vergeudung von Blutkonserven vermieden, welche dadurch hervorgerufen wird, daß z.B. angebrauchte Blutkonserven oder für einen Einsatz vorbereitete Blutkonserven nicht lange genug gelagert werden konnten, sodaß sie nach Ablauf der Lagerzeit von 6 Stunden verworfen werden mußten.

## Patentansprüche

1. Mehrfachbeutelsystem zur sterilen Behandlung von menschlichen Blutkonserven, mit dessen Hilfe das Blut filtriert und gewaschen werden kann, und wobei wenigstens ein Auffangbeutel für nicht benötigte Bestandteile mit dem Blutsammelbeutel verbunden ist, dadurch gekennzeichnet, daß der Blutsammelbeutel an einem Ende einer Verteilerleitung (3) und ein Vorratsbeutel (4) für die Waschflüssigkeit an deren anderem Ende steril angeschlossen ist, wobei die Verteilerleitung (3) vor dem Vorratsbeutel (4), vorzugsweise mittels einer Klemme (5) od.dgl., abschließbei ist, und wobei in bekannter Weise von der Verteilerleitung (3) wenigstens zwei parallel verlaufende Abzweigleitungen (6, 7) ausgehen, daß eine davon zu einem Auffangbeutel (11) für verbrauchte Waschflüssigkeit und die andere zu einem Aufnahmebeutel (10) für gewaschenes Erythrozytenkonzentrat führt und daß weiters in bekannter Weise in der Verteilerleitung (3) dem Blutsammelbeutel (1) ein Leukozytenfilter (2) nachgeschaltet ist.

2. Mehrfachbeutelsystem nach Anspruch 1, dadurch gekennzeichnet, daß von der Verteilerleitung (3) wenigstens eine weitere Abzweigungsleitung (8, 9) ausgeht, an die eine oder mehrere weitere Auffangbeutel (12) für verbrauchte Waschflüssigkeit und/oder Vorratsbeutel (13) für weitere Behandlungsflüssigkeiten angeschlossen sind.

3. Mehrfachbeutelsystem nach einem der Ansprüche 1 oder 2, dadurch gekennzeichnet, daß die Auffangbeutel (11, 12) für verbrauchte Waschflüssigkeit und/oder Vorratsbeutel (13) für weitere Behandlungsflüssigkeiten, z.B. einer speziellen Erythrozytennährlösung, über an sich bekannte abbrechbare Ventile (14, 15, 16) gegenüber der Verteilungsleitung (3) abgeschlossen und durch Wegbrechen des Ventils öffenbarsind.
